# EUROPEAN PATENT SPECIFICATION

(11) **EP 4 593 775 B1**
(45) Date of publication and mention of the grant of the patent: **09.12.2026**
(21) Application number: 23792870.0
(22) Date of filing: 14.09.2023
(51) Int. Cl.: A61F 13/494, A61F 13/495, A61F 13/49, A61F 13/537, A61F 13/496

(54) **PANT-TYPE ABSORBENT ARTICLE WITH COMPOSITE ISOLATION SHEET**
HOSENARTIGER SAUGFÄHIGER ARTIKEL MIT VERBUNDISOLIERFOLIE
ARTICLE ABSORBANT DE TYPE CULOTTE AVEC FEUILLE D'ISOLATION COMPOSITE

(30) Priority: 28.09.2022 WO PCT/CN2022/122097; 24.10.2022 WO PCT/CN2022/126978
(43) Date of publication of application: 06.08.2025
(73) Proprietor: The Procter & Gamble Company, Cincinnati, OH 45202 (US)
(72) Inventor: YUAN, Yi, Beijing 101312 (CN); ZHU, Tianzhuo, Beijing 101312 (CN); ZHANG, Jing, Beijing 101312 (CN); MORIMOTO, Koichi, Cincinnati, Ohio 45202 (US); CHEN, Lei, Beijing 101312 (CN)
(74) Representative: P&G Patent Germany
(86) International application number: PCT/CN2023/118883
(87) International publication number: WO 2024/067136

(56) References cited:
- EP-A1- 1 297 809
- EP-A1- 1 417 947
- WO-A2-94/28844
- GB-A- 2 287 888
- US-A- 4 662 877

## Description

### FIELD OF THE INVENTION

This invention relates to pant-type absorbent articles having a composite isolation sheet for isolating body exudates from the wearer's skin.

### BACKGROUND OF THE INVENTION

Absorbent articles for personal hygiene, such as disposable diapers, disposable pants, and adult incontinence undergarments, are designed to absorb and contain various body exudates, including urine, menses, low viscosity fecal matter, and solid fecal matter. Urine may be difficult to quickly isolate and contain while the wearer is sleeping, and particularly when the wearer is sleeping on one's side. Moreover, it is known that when fecal material and urine meet on the skin, this may cause irritation and redness of the skin and sometimes even dermatitis of the skin.

Absorbent articles with means to isolate urine have been known in the art, such as in JP Publication 2001-252302 A, US 4 662 877, GB 2287888 A, WO94/28844 A2, EP 1 297 809 A1 and EP 1 417 947 A1. While such absorbent articles have been known, they have shortcomings that have prevented significant commercial success. For example, those known in the art may have a front side isolation sheet with an edge extending straight in the transverse direction, or may have elastic strands at the edge of the front side isolation sheet. Such configurations for the front side isolation sheet may cause irritation to the wearer's genital area.

Based on the foregoing, there is a need for an absorbent article having an isolation sheet on the front side for preventing urine leakage while maintaining the performance of containment and wear comfort. There is also a need for such an absorbent article which may be economically manufactured.

### SUMMARY OF THE INVENTION

The present invention is directed to an absorbent article having a longitudinal direction and a transverse direction comprising a front elastic belt, a back elastic belt, and a crotch region, wherein the front and back elastic belts are connected with a pair of side seams to form a waist opening and a pair of leg openings; the crotch region extending longitudinally between the front elastic belt and the back elastic belt; the absorbent article comprising an absorbent main body extending the entire longitudinal dimension of the crotch region and further extending partly into each of the front region and the back region, the absorbent main body comprising:
1) a water permeable tipsheet,
2) a water impermeable backsheet,
3) an absorbent core disposed between the topsheet and the backsheet, and
4) a composite isolation sheet comprising:
   a) a pair of inner cuffs having a pair of side edges which are elasticized in the longitudinal direction with an inner cuff elastic strand, and
   b) a front portion, the front portion having a front longitudinal end point in the front region and a pair of front opening edges wherein the front opening edges are free of elastic elements, wherein the front portion has a side longitudinal dimension F1, and a center longitudinal dimension F2 wherein F2 is from about 30% to about 85% of F1,

wherein the composite isolation sheet is bonded to the wearer facing side of the topsheet by a pair of main bonds extending in the longitudinal direction and by an auxiliary bond extending in the transverse direction between the main bonds,
the front elastic belt formed by an outer sheet, an inner sheet, and a plurality of elastic strands extending in the transverse direction and sandwiched between the outer sheet and the inner sheet, the front elastic belt comprising a transfer elastic strand which is at least one elastic strand superposing the front portion as well as the pair of main bonds,
wherein the absorbent core does not extend to the transverse edges beyond the main bonds, and does not extend to the front end beyond the auxiliary bond.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1 is a schematic perspective view of an embodiment of an absorbent article of the present invention.
Figures 2A-2C are schematic plan views of embodiments of absorbent articles of the present invention with the seams removed and in its flat uncontracted condition showing the wearer-facing surface.
Figure 3 is a chart showing the relationship of T1 and F1 for the composite isolation sheet of the present invention.
Figure 4A is a schematic plan view of Figure 2A showing the elastics of the front elastic belt underneath the absorbent main body.
Figure 4B is a schematic plan view of Figure 2C showing the elastics of the front elastic belt underneath the absorbent main body.
Figures 4C-4F are schematic plan views of the composite isolation sheet of the present invention.
Figures 5A-5D are schematic cross section views of the composite isolation sheet of Figure 4A taken at line A-A.
Figure 6A is a schematic cross section view of the front region of Figure 2A taken at LX with the elastic strands abbreviated.
Figure 6B is a schematic cross section view of the back region of Figure 2A taken at LX with the elastic strands abbreviated.
Figure 6C is a schematic cross section view of the back region of Figure 2B taken at LX with the elastic strands abbreviated.
Figures 7A-7B are schematic plan views of the back region of Figure 2B with the back portion abbreviated.
Figure 7C is a schematic cross section view of the back region of Figure 2B taken at line B-B with the back portion abbreviated.
Figure 8 is a schematic view of an example of a hanger-type sample holding fixture according to the "Whole Article Force Measurement" herein.

### DEFINITIONS

As used herein, the following terms shall have the meaning specified thereafter:
"Absorbent article" refers to articles of wear which may be in the form of pants, taped diapers, incontinent briefs, feminine hygiene garments, and the like, so configured to also absorb and contain various exudates such as urine, feces, and menses discharged from the body. The "absorbent article" may serve as an outer cover adaptable to be joined with a separable disposable absorbent insert for providing absorbent and containment function, such as those disclosed in PCT publication WO 2011/087503A.

"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Proximal" and "distal" refer respectively to the position closer or farther relative to the longitudinal center of the article.

"Wearer-facing" and "garment-facing" refer respectively to the relative location of an element or a surface of an element or group of elements. "Wearer-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m² or gsm).

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material is considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material is considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Dimension", "Length", "Width", "Pitch", "Diameter", "Aspect Ratio", "Angle", and "Area" of the article are all measured in a state wherein the article is extended to the Full Stretch Circumference W1 according to the "Whole Article Force Measurement" herein, and utilizing a ruler or a loupe, unless specified otherwise.

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to 0 ±2, and a b* value equal to 0 ±2 according to the CIE L* a* b* color system.

### DETAILED DESCRIPTION OF THE INVENTION

### Absorbent Article

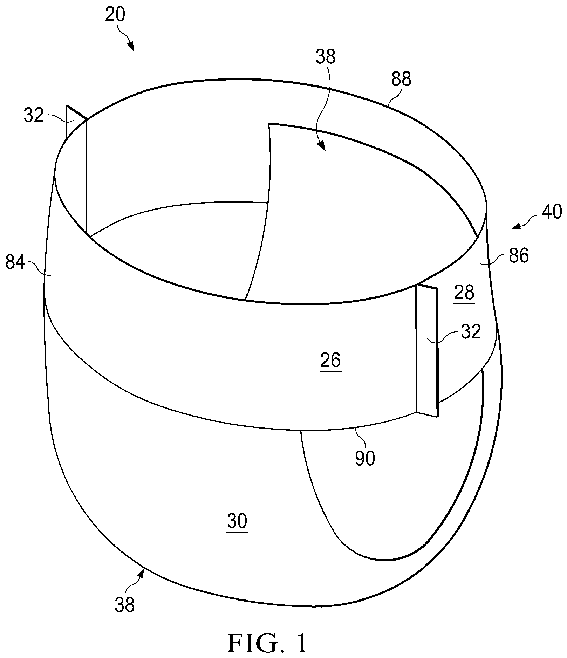

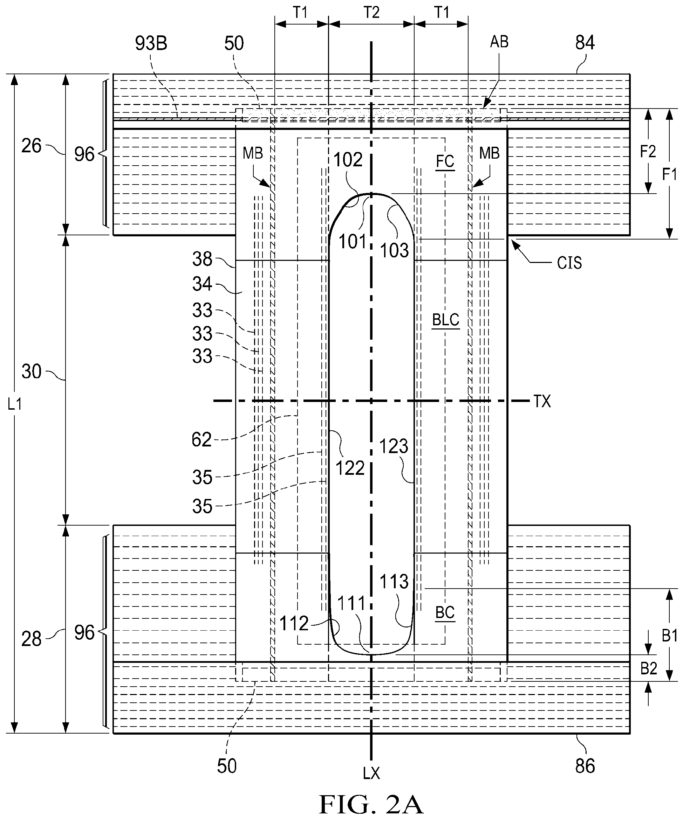

Figure 1 is a perspective view of an absorbent article (20) of the present invention, and Figure 2A is a schematic plan view of an absorbent article with the seams removed and in its flat uncontracted condition showing the wearer-facing surface. The absorbent article (20) has a longitudinal centerline LX which also serves as the longitudinal axis, and a transverse centerline TX which also serves as the transverse axis. The absorbent article (20) has a wearer facing surface, a garment facing surface, a front elastic belt (84), a back elastic belt (86), a crotch region (30), and side seams (32) which join the front elastic belt (84) and the back elastic belt (86), to form two leg openings and a waist opening.

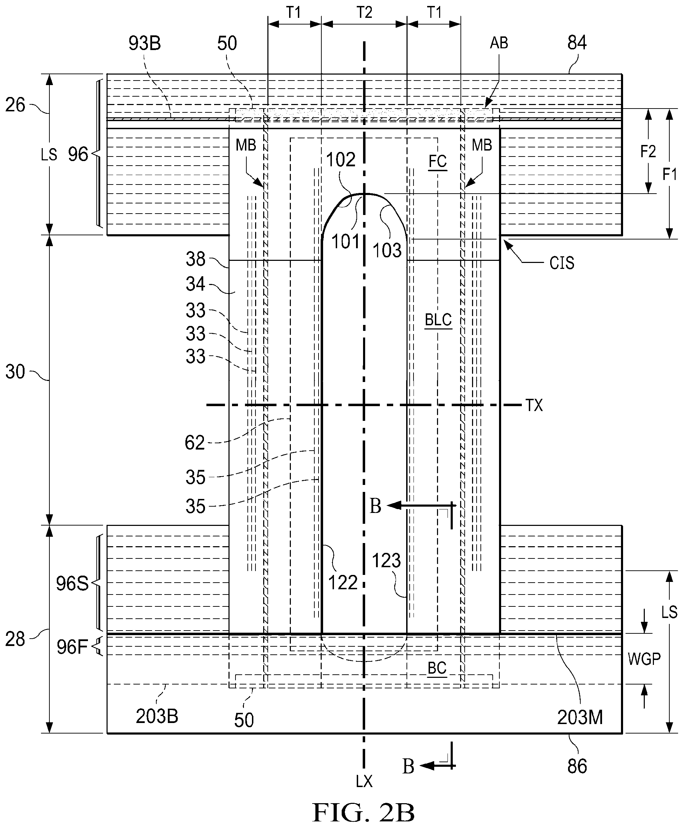

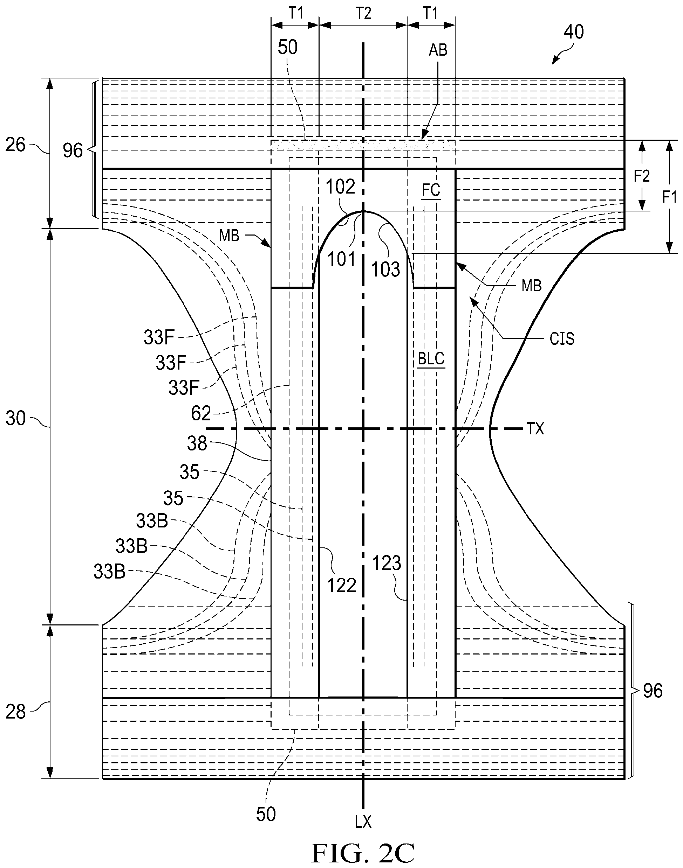

The absorbent article (20) may be a belt-type pant as in Figures 1, 2A and 2B comprising an absorbent main body (38) to cover the crotch region (30) of the wearer, a front elastic belt (84) and a back elastic belt (86) (hereinafter may be referred to as "front and back elastic belts"), the front and back elastic belts (84, 86) forming a discrete ring-like elastic belt (40) extending transversely defining the waist opening. For the belt-type pant, the discrete ring-like elastic belt (40) may also be referred to as the elastic belt (40). For the belt-type pant as in Figures 2A and 2B, the front and back elastic belts (84, 86) and the absorbent main body (38) jointly define the leg openings. For the belt-type pant, the front elastic belt (84) is the front region (26), and the back elastic belt (86) is the back region (28), and the remainder is the crotch region (30). Referring to Figure 2C, the absorbent article (20) may be a uni-body type pant configured such that an elastic cover (40) forms the garment facing surface of the entire article. For the uni-body type pant, the portion extending in the transverse direction between the side seams (32), respectively, are considered the front region (26) and the back region (28), and the remainder is the crotch region (30). For the uni-body type pant, the front region (26) is considered the front elastic belt (84), and the back region (28) is considered the back elastic belt (86).

The front and back elastic belts (84, 86) of the article of the present invention acts to dynamically create fitment forces and to distribute the forces dynamically generated during wear. The front and back elastic belts (84, 86) are joined with each other outside the side edges (89) to form side seams (32), a waist opening and two leg openings. Each leg opening may be provided with elasticity around the perimeter of the leg opening. The elasticity around the leg opening may be provided by the leg elastic bodies (33F, 33B) provided along the shaped crotch region of the elastic cover (40) as in Figure 2C, or by the combination of elasticity from the front elastic belt (84), the back elastic belt (86), and the gasketing leg cuffs (34) of the absorbent main body (38) as in Figures 1, 2A, and 2B.

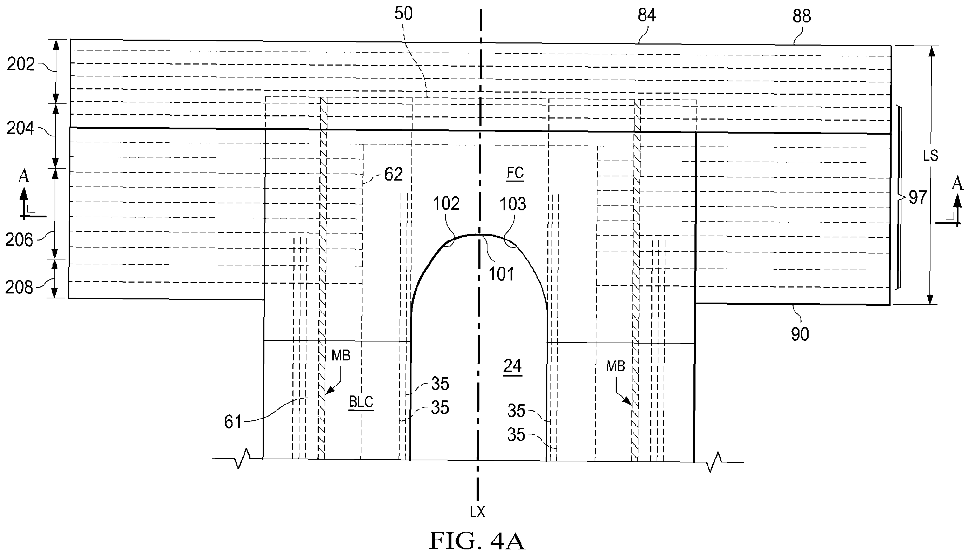

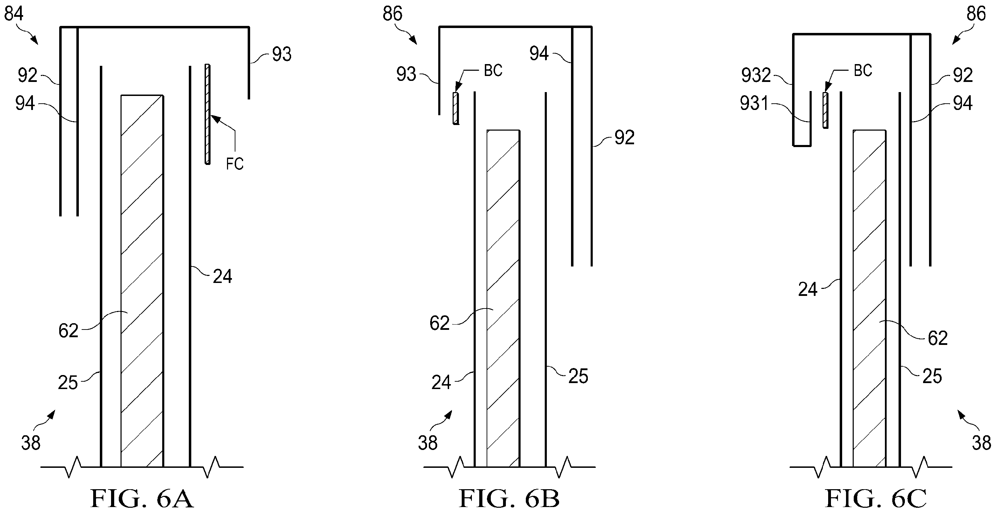

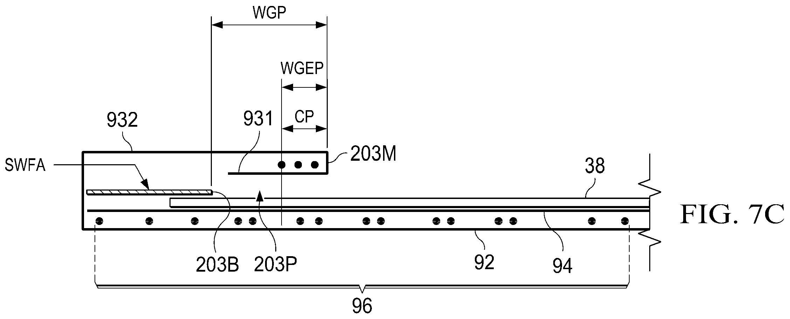

The front elastic belt (84) and back elastic belt (86) are configured to impart elasticity in the transverse direction. Referring to Figures 6A-6C and 7C, the front elastic belt (84) and the back elastic belt (86) may each be formed by an inner sheet (94), an outer sheet (92), and a plurality of elastic members (96) running in the transverse direction, and a fold over (93) wherein the fold over (93) is an extension of the inner sheet or outer sheet material formed by folding the inner sheet or outer sheet material at the distal edge (88) of the front and back elastic belts; wherein the belt elastic members (96) are sandwiched between two of these sheets. There may be more than one fold over (931, 932) as discussed in further detail below. Referring to Figures 2A-2C, at least some of the elastic members (96) extend in the transverse direction substantially parallel to each other. All of the elastic members (96) may extend in the transverse direction substantially parallel to each other. Referring to Figures 4A-4B, the elastic members (96) of the front elastic belt (84) which exist in active elasticity underneath the absorbent main body (38) are shown. At least 10%, or at least from about 15% to not more than about 70%, of the front and back elastic belts (84, 86) from the waist opening in the longitudinal direction may be a laminate in active elasticity along the entire transverse dimension of the front and back elastic belts (84, 86). For each front and back elastic belt (84, 86), the region overlapping the absorbent core (62) may be removed of its elastic activity. Such region removed of elastic activity is referred herein as the "elastic cut window", and the remainder of the intact elastic member capable of imparting elasticity is defined as the "effective length of elasticity of an elastic member".

The tensile stress (N/m) of the entirety of the front and back elastic belts (84, 86), respectively, may be profiled in order to provide the functional benefits of the present invention, such as ease of stretch and application, while also maintaining certain force during wear, to prevent the article from sagging after loading. When the elasticity of the front and back elastic belts (84, 86) are provided by a plurality of elastic members (96) running in the transverse direction, the tensile stress may be adjusted by one or more of the following methods; 1) elongation rate of the elastic member (96); 2) density (dtex) of the elastic member (96); 3) longitudinal pitch of multiple elastic members (96); and 4) effective length of elasticity of the elastic member (96) in the transverse direction. By elongation, "0% elongation" is meant the original length of the elastic member.

### Absorbent Main Body

The absorbent main body (38) of the present invention comprises a water permeable topsheet (24), a water impermeable backsheet (25) and an absorbent core (62) disposed between the topsheet (24) and the backsheet (25). The topsheet (24) may be a water permeable substrate. The backsheet (25) may be a water impermeable film. Referring to Figures 2A-2C and 4A-4B, the absorbent main body (38) comprises an absorbent core (62) for absorbing and containing body exudates disposed on the absorbent main body (38), and an absorbent material non-existing region (61) surrounding the periphery of the absorbent core (62). The absorbent material non-existing region (61) may be made of material selected from the group consisting of the topsheet (24), the backsheet (25), the material forming the composite isolation sheet (CIS) extending outwardly, cuff material, the optional outer cover layer, and any combination thereof. In the embodiments of Figures 2A-2C, the absorbent main body (38) has a generally rectangular shape, left and right longitudinally extending side edges and front and back transversely extending end edges (50). The absorbent main body (38) extends the entire longitudinal dimension of the crotch region (30) and further extends partly into each of the front region (26) and the back region (28).

### Topsheet and Backsheet

The absorbent main body (38) of the present invention comprises a water permeable topsheet (24) that may be positioned at least in partial contact or close proximity to a wearer. Suitable top sheets (24) may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet (24) is generally supple, soft feeling, and non-irritating to a wearer's skin. The topsheet (24) is water permeable, permitting bodily fluids to readily penetrate through the thickness of the topsheet (24). One topsheet (24) useful herein is available from Fibertex NiLai, Malaysia with tradename H30501221 or FQN Hazlet NJ with tradename SB1206169. Any portion of the topsheet (24) may be coated with a lotion or skin care composition as is known in the art. Portions of the topsheet (24) that do not overlap the composite isolation sheet may be coated with a lotion. Examples of suitable lotions include those described in U.S. Patent Nos. 5,607,760; 5,609,587; 5,635,191; and 5,643,588.

The absorbent main body (38) of the present invention comprises a water impermeable backsheet (25) which is designed to prevent the exudates absorbed by and contained within the absorbent core (62) from soiling articles that may contact the absorbent article, such as bed sheets and undergarments. The backsheet (25) may be positioned such that it extends beyond the absorbent core (62) in both the longitudinal direction and the transverse direction. Suitable backsheet (25) materials include films such as those manufactured by Plaster Argentina with tradename PLBA NBBS 10-12GSM PR V1. Other suitable backsheet (25) materials may include breathable materials that permit vapors to escape from the absorbent article while still preventing exudates from passing through the backsheet (25). Exemplary breathable materials may include materials such as woven webs, nonwoven webs, composite materials such as film-coated nonwoven webs, and microporous films such as manufactured by Daika Japan with tradename MPF DKH-180 15G V7 and manufactured by Berry Nashville, TN with trademark BR-137P V13. Such breathable composite materials are described in greater detail in PCT Application No. WO 95/16746 and U.S. Patent No. 5,865,823. Other breathable backsheets including nonwoven webs and apertured formed films are described in U.S. Patent No. 5,571,096. An exemplary, suitable backsheet is disclosed in U.S. Patent No. 6,107,537. Other suitable materials and/or manufacturing techniques may be used to provide a suitable backsheet including, but not limited to, surface treatments, particular film selections and processing, particular filament selections and processing, etc.

### Absorbent Core

The absorbent main body (38) of the present invention comprises an absorbent core (62) for absorbing and containing body exudates disposed on the wearer facing side. The absorbent core (62) may include an absorbent layer and an acquisition system. The absorbent layer is the region wherein absorbent materials having a high retention capacity, such as superabsorbent polymers, are present. The absorbent core (62) may exist through the entire longitudinal dimension of the crotch region and extending at least partly in the front region (26); or at least partly in both the front and back regions (26, 28). The absorbent layer may be substantially cellulose free. Alternatively, the absorbent layer may contain cellulose. There may be an absorbent layer mainly comprising cellulose, and another absorbent layer mainly comprising superabsorbent polymers.

Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multiconstituent fibers such as side by side, core/sheath or island in the sea type fibers. Such synthetic fibers may be formed via a spunbonding process or a meltblowing process.

The absorbent core (62) may also include an acquisition system for facilitating the acquisition and distribution of body exudates, and may be placed between the topsheet (24) and the absorbent layer. The acquisition system may include cellulosic fibers. The absorbent layers may be disposed in plurality in the absorbent core (62). Some portions of the absorbent layers may be configured to have substantially no absorbent material to form a channel or a plurality of channels. Channels may be useful for allowing the absorbent core (62) to bend upon swelling with fluids, such that the center region conforms to the wearer's body after swelling and prevent sagging of the article. The channels may also be formed in the acquisition system, and may be configured to at least partly match the channels of the absorbent layer in the thickness direction. The acquisition system may comprise a liquid management layer directly under the topsheet (24). The function of such a layer is to rapidly acquire the fluid from the topsheet (24) away from the wearer-facing side and/or to distribute over a larger area so it is more efficiently absorbed by the absorbent core. It is also possible that such a liquid management layer may be placed between the backsheet (25) and the absorbent core. The liquid management layer may be a spunlace nonwoven comprising viscose, PET, CoPET/PET fibers, and combinations thereof.

The absorbent core (62) may comprise a high loft material encompassing superabsorbent polymers. The term "high loft" refers to low density bulky fabrics, as compared to flat, paper-like fabrics. High loft webs are characterized by a relatively high porosity. This means that there is a relatively high amount of void space in which superabsorbent polymer particles can be distributed. The high loft material (without the superabsorbent particles) of the invention may have a density at a pressure of 4. 14kPa (0.6 psi) below 0.20 g/cm³ in particular ranging from 0.05 g/cm³to 0.15 g/cm³. The high loft layer (without the superabsorbent particles) may have a density at a pressure of 2.07 kPa (0.3 psi) below 0.20 g/cm³ in particular ranging from 0.02 g/cm³to 0.15 g/cm³. The high loft layer (without the superabsorbent particles) of the invention may have a density at a pressure of 0.83 kPa (0.12 psi) below 0.15 g/cm³ in particular ranging from 0.01 g/cm³to 0.15 g/cm³, and a basis weight of from 15 to 500gsm, preferably 30~200gsm, such as those described in US 2021/0361497 A1. The absorbent core (62) comprising high loft material encompassing superabsorbent polymers may also contain channels.

Alternatively, the absorbent core (62) may comprise an absorbent layer having superabsorbent polymers disposed between first and second layers of nonwoven material immobilized by a fibrous layer of thermoplastic adhesive material (not shown). The first and second layers of nonwoven materials may be relatively low basis weight nonwoven fibrous webs including synthetic fibers, such as mono-constituent fibers of PE, PET and PP, multiconstituent fibers such as side by side, core/sheath or island in the sea type fibers. Such synthetic fibers may be formed via a spunbonding process or a meltblowing process.

### Composite Isolation Sheet

Referring to Figures 2A-2C, the absorbent main body (38) of the present invention comprises a composite isolation sheet (CIS) bonded to the wearer facing side of the topsheet (24) by a pair of main bonds (MB) extending in the longitudinal direction, for isolating body exudates and particularly urine from the wearer's skin. The composite isolation sheet (CIS) comprises a) a pair of inner cuffs (BLC) having a pair of side edges which are elasticized in the longitudinal direction, b) a front portion (FC), and c) an optional back portion (BC). The region and area of the composite isolation sheet (CIS) is defined by the front end of the front portion, the back end of the inner cuffs (BLC) or optional back portion (BC), and the pair of main bonds (MB). The longitudinal and transverse center of the composite isolation sheet (CIS) is open for allowing body exudates to be contained in the absorbent core (62) through the topsheet (24). The opening of the composite isolation sheet (CIS) is defined by the inner cuff side edges (122, 123), the front opening edges (102, 103) and either the optional back opening edges (112, 113) or the fold over (93) of the back elastic belt (86), as detailed below.

In the embodiments of Figures 2A-2C, the front end of the front portion (FC) matches the front end of the absorbent main body (38). In a typical manufacturing process, elements of the composite isolation sheet (CIS) are formed as a continuous material bonded to the remainder of the continuous absorbent main body, and then cut into individual absorbent main bodies (38). The main bonds (MB) may be formed by adhesive, heat, ultrasonic, pressure, or combinations thereof, preferably heat and pressure. Although the substrate for providing the composite isolation sheet (CIS) may extend into other elements of the absorbent main body (38) such as the outer cuffs as described later, such other elements are not considered the composite isolation sheet (CIS). Referring to Figures 2A-2B, the main bonds (MB) may be provided on the wearer facing side of the absorbent main body (38) leaving some remaining transverse dimension of the absorbent main body outward of the main bonds (MB). Referring to Figure 2C, the material for forming the composite isolation sheet (CIS) may be folded against the transverse side edges of the absorbent main body (38) towards the garment facing side of the absorbent main body, while the main bonds (MB) may be provided at the transverse side edges of the absorbent main body (MB).

In addition to the main bonds (MB) extending in the longitudinal direction, the composite isolation sheet (CIS) is bonded to the wearer facing side of the topsheet (24) by an auxiliary bond (AB) extending in the transverse direction between the main bonds (MB). The auxiliary bond (AB) may be provided only in a dimension between the main bonds (MB), or may be provided for the entire transverse dimension of the absorbent main body (38). The auxiliary bond (AB) may also be formed by adhesive, heat, ultrasonic, pressure, or combinations thereof. The auxiliary bond (AB) may be continuous along its entire dimension, or may be provided discontinuous, such as in stripes spaced apart in the transverse direction. The auxiliary bond (AB) may be continuous for providing secure prevention of leakage beyond the absorbent main body (38). The auxiliary bond (AB) may be discontinuous for preventing stiffness in this region. When the auxiliary bond (AB) is provided in stripes spaced apart in the transverse direction, each spacing is no greater than 10mm. The absorbent core (62) does not extend to the transverse edges beyond the main bonds (MB), and does not extend to the front end beyond the auxiliary bond (AB). By disposing the absorbent core (62) within the main bonds (MB) and auxiliary bonds (AB) this helps exudates to be effectively contained in the absorbent core (62).

Referring to Figures 2A-2C and 4A-4B, the composite isolation sheet (CIS) comprises a pair of inner cuffs (BLC) extending for the remainder of the longitudinal length of the absorbent main body (38) except for the front portion (FC) or the optional back portion (BC), and forms a pair of side edges which are elasticized in the longitudinal direction along the longitudinally extending side edges (122, 123). The pair of side edges (122, 123) are elasticized at least in the crotch region (30), and may be devoid of elasticity adjacent the front end and/or back end of the absorbent main body (38). The crossing point of the front portion (FC) with the side edges (122, 123) may be in active elasticity. Referring to Figure 2A, the crossing point of the optional back portion (BC) and the side edges (122, 123) may be in active elasticity. Referring to Figures 2A-C and 4A-B, the pair of side edges (122, 123) of the inner cuffs (BLC) are elasticized by longitudinally extending elastic bodies (35) disposed adjacent the side edges (122, 123). The elastic bodies for elasticizing the side edges (122, 123) may be provided in a plurality of elastic strands (35). The inner cuff elastic strands (35) in active elasticity may extend into the front portion (FC) as well as the optional back portion (BC). The inner cuff (BLC) may have a transverse dimension (T1) between the main bond and the closest side edge (122, 123) of from about 25mm to about 50mm. Such transverse dimension may be the same for the entire composite isolation sheet (CIS). The transverse distance (T2) between the side edges (122, 123), when the inner cuffs (BLC) are completely laid flat on the topsheet (24), may be from about 50mm to about 140mm.

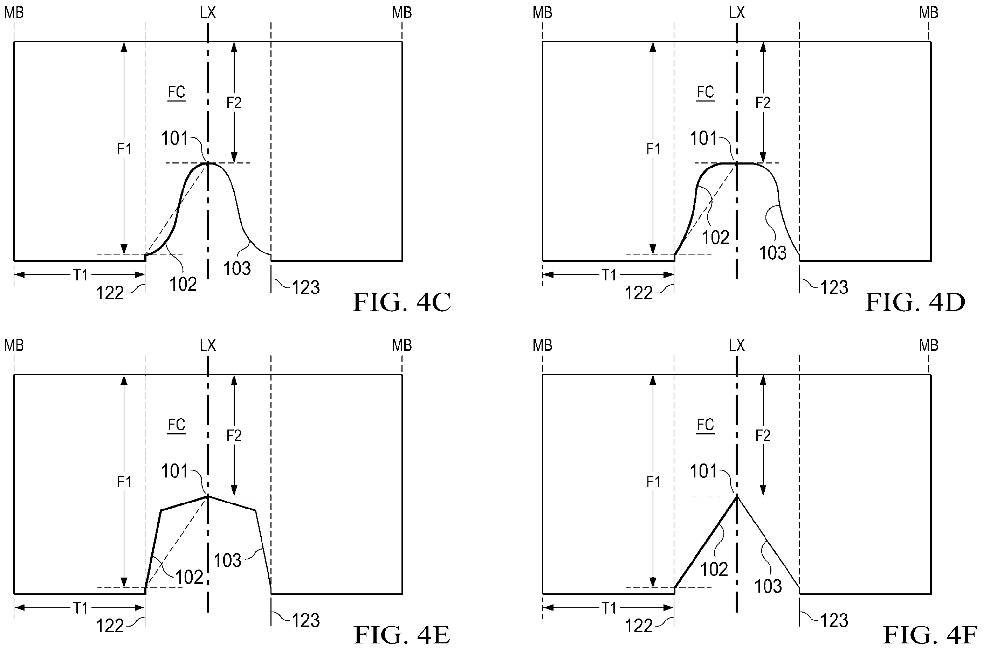

Referring to Figures 2A-C and 4A-B, the composite isolation sheet (CIS) comprises a front portion (FC) defined by the line extending parallel to the transverse axis matching the crossing point of the transverse element of the opening on the front side and the pair of inner cuffs (BLC), the front transversely extending end edge (50) of the absorbent main body (38), and the main bonds (MB). The front portion (FC) of the composite isolation sheet (CIS) upon wear does not cover the urethral orifice of the wearer. The front portion (FC) has a front longitudinal end point (101) and a pair of front opening edges (102, 103), wherein the front opening edges (102, 103) are so configured that the front longitudinal end point (101) is positioned on the longitudinal axis and within the front region (26). The pair of front opening edges (102, 103) may be in the front region (26), and may extend from the front region (26) into the crotch region (30). The front opening edges (102, 103) are not elasticized. The front portion (FC) has a side longitudinal dimension (F1) which may be from about 8% to about 50%, or from about 22% to about 50% of the longitudinal dimension (L1) of the absorbent main body (38). The front portion (FC) has a center longitudinal dimension (F2) between the front longitudinal end point (101) to the front end of the absorbent main body. Dimension F2 is from about 5% to about 95%, preferably from about 30% to about 85% of dimension F1. The front opening edges (102, 103) are line symmetrical about the longitudinal axis, and may be curved, straight, or formed by a series of straight lines. Referring to Figures 4C-4E, the front opening edges (102, 103) may comprise a concave portion against the front portion. Referring to Figure 4C, the front opening edges (102, 103) may form a portion of a sinusoidal curve. Referring to Figure 4D, the front opening edges (102, 103) together may form a portion of a circle or an oval. Referring to Figure 4E, the front opening edges (102, 103) together may be a series of straight lines. Referring to Figure 4F, the front opening edges (102, 103) may be straight lines.

Referring to Figure 2A, when the composite isolation sheet (CIS) comprises a back portion (BC), the back portion (BC) is defined by the line extending parallel to the transverse axis matching the crossing point of the transverse element of the opening on the back side and the pair of inner cuffs (BLC), the back transversely extending end edge (50) of the absorbent main body, and the main bonds (MB). When the composite isolation sheet (CIS) comprises a back portion (BC), the composite isolation sheet (CIS) upon wear does not cover the anal orifice of the wearer. Still referring to Figure 2A, when the composite isolation sheet (CIS) comprises a back portion (BC), the back portion (BC) has a back longitudinal end point (111) and a pair of back opening edges (112, 113), wherein the back opening edges (112, 113) are so configured that the back longitudinal end point (111) is positioned on the longitudinal axis and optionally within the back region (28). The back opening edges (112, 113) may be curved, straight, or formed by a series of straight lines. The back opening edges (112, 113) are not elasticized. The back portion (BC) has a side longitudinal dimension (B1) which may be from about 5% to about 35% of the longitudinal dimension of the absorbent main body (L1). The back portion (BC) has a center longitudinal dimension (B2) between the back longitudinal end point (111) to the back end of the absorbent main body. Dimension B2 is from about 5% to about 95%, preferably from about 30% to about 85% of dimension B1. Referring to Figure 2B, when the composite isolation sheet (CIS) is either devoid of a back portion (BC) or comprises a back portion (BC) that does not extend beyond the proximal edge of the fold over (93), the back side of the opening of the composite isolation sheet (CIS) is defined by proximal edge of the fold over (93) of the back elastic belt (86).

The composite isolation sheet of the present invention may have T1 (mm) and F1 (mm) in the relationship of: $F 1 \geq 248.14 - 4.66 \times T 1 + 0.34 \times {\left(T1-40.79\right)}^{2} .$

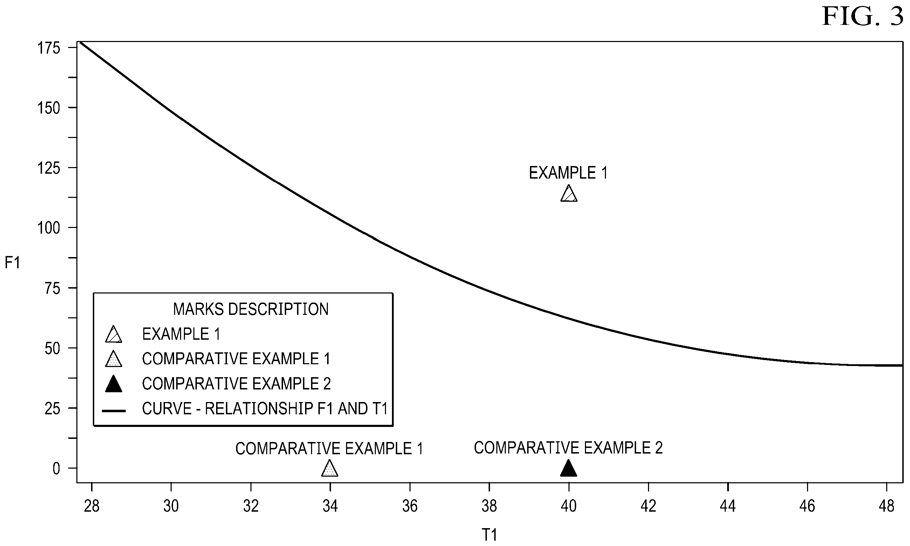

Such relationship is described in Figure 3 as the region above the curvature. Without being bound by theory, by providing T1 and F1 to have the relationship as above while also providing F2 to be from about 30% to about 85% of F1, good containment of urine is provided without causing irritation to the wearer's genital area.

Material for making the composite isolation sheet (CIS) may be substantially water impervious material. The material may be an SMS nonwoven or an SMMS nonwoven material, or a nonwoven component layer comprising fine fibers having an average diameter of less than 1 micron. One useful combination of nonwoven fabric webs may include spunbond, meltblown, spunbond ("SMS") webs comprising outer layers of spunbond thermoplastics (e.g., polyolefins) and an interior layer of meltblown thermoplastics. Suitable composite isolation sheet material useful herein include those of SMS type available from Toray Polytech Nantong China with tradename LIVSEN SMS 13, available from FQN Hazlet NJ with tradename SM15009270, and available from Fibertex Aalborg Denmark with tradename B10160HS. The material may be treated, by region or in part of a region, with a lotion or a hydrophobic surface coating to provide various physical properties. Material for making the composite isolation sheet (CIS) may have a hydrostatic head of greater than about 2mbar, or greater than about 3mbar, or greater than about 4mbar. The material may have a hydrostatic head of less than about 200mbar, or less than about 100mbar, or less than about 75mbar, or less than about 50mbar, or less than about 25mbar, or less than about 15mbar. The material may have an opacity of from about 15% to about 50% hunter opacity, or from about 20% to about 45% hunter opacity. The material may have an opacity of from about 45% to about 75% hunter opacity; or from about 50% to about 70% hunter opacity. The material may have an air permeability of less than about 50 m³/m²/min; or less than about 45 m³/m²/min. The material may have an air permeability of greater than about 5 m³/m²/min; or greater than about 10 m³/m²/min; or greater than about 15 m³/m²/min; or greater than about 20 m³/m²/min.

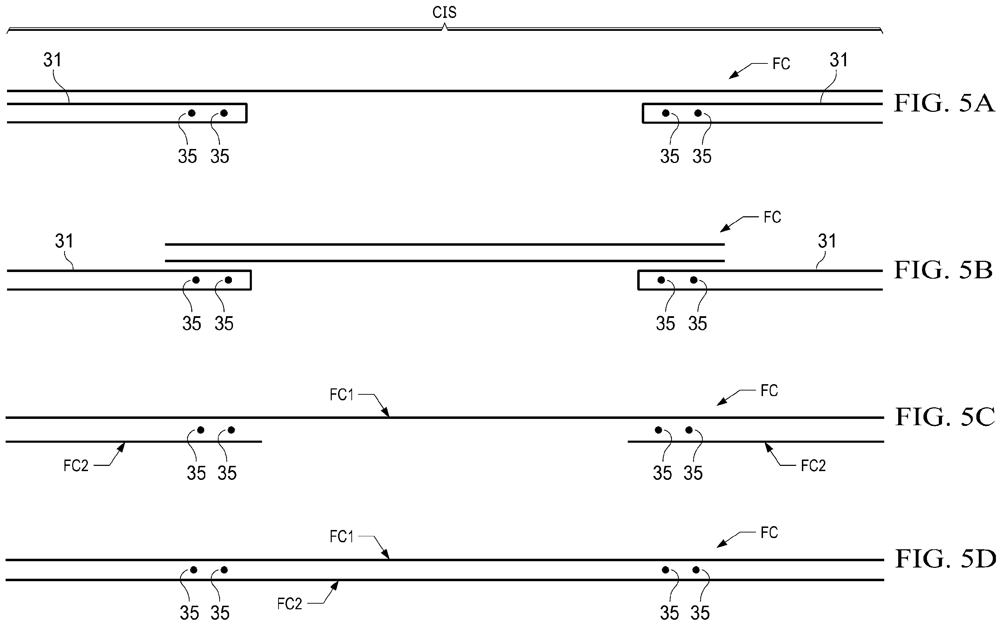

Referring to Figures 5A- 5D, various configurations for forming the composite isolation sheet (CIS) are disclosed. Figures 5A - 5D are schematic cross section views taken at line A-A of Figure 4A, with the substrates for making the composite isolating sheet (CIS) extended laterally and detached from the remainder of the absorbent main body (38).

Figures 5A - 5B are related to configurations wherein the inner cuff (BLC) is formed by a different part from the front portion (FC). The material for forming the inner cuff (BLC) may be the same or different from the material for forming the front portion (FC). In these configurations, the inner cuff (BLC) is formed by an inner cuff part (31) extending the entire length of the absorbent main body (38). When taking such configuration for the inner cuff (BLC), at least the center region (30) is in active elasticity. When taking such configuration for the inner cuff (BLC), the inner cuff part (31) overlapping the front portion (FC) may be devoid of active elasticity, particularly along the front end (50) of the absorbent main body (38). Still referring to Figures 5A - 5B, the front portion (FC) is formed by a front part that is bonded on the wearer-facing side of the inner cuff part (31). Referring to Figure 5A, the front part may be provided in one layer, and extending for the entire transverse dimension between the main bonds (MB). Referring to Figure 5B, the front part may be provided in 2 layers, wherein each layer provides different functions, for example the wearer-facing layer may be provided with material having soft touch to the skin, while the other layer may be provided with material having low hydrostatic head. As in Figure 5B, the transverse dimension of the front portion (FC) may be smaller than that of the inner cuff (BLC).

Figures 5C - 5D are related to configurations wherein the inner cuff (BLC) and the front portion (FC) are formed by the same integral part. In these configurations, the elasticizing of the side edges of the inner cuff (BLC) may be provided by the outer front portion sheet (FC1) and the inner front portion sheet (FC2). Referring to Figure 5C, the front part may be provided in one layer. Referring to Figure 5D, the front part may be provided in 2 layers.

For any of the configurations of Figures 5A - 5D, the optional back portion (BC) may be formed in the same way as the front portion (FC) as described above.

In some configurations such as in Figure 2B, the back portion (BC) may be provided in a dimension relatively smaller than the front portion (FC) and relatively smaller than the waist guard portion (WGP) which is discussed in further detail below. There is benefit in having even a small area of back portion (BC) for process feasibility. Prior to assembling with the remainder of the absorbent main body (38), the front portion (FC) and back portion (BC) may be formed by a continuous material wherein the front and back opening edges (102, 103, 112, 113) are collectively formed by a die cutter. The die cut continuous material then may be bonded to a continuous absorbent main body in a position bridging the front end and back end of the continuous absorbent main body, and then finally cut into individual absorbent main bodies. By providing die cut to the back portion (BC) together with the front portion (FC) this enables distribution of pressure provided by the die cutter against the continuous material.

### Front Portion and Front Elastic Belt

In the absorbent article of the present invention, the front portion (FC) and components of the front elastic belt (84) have a particular relationship in order for the front portion (FC) to function as intended. Referring to Figure 4A, the front elastic belt (84) comprises a transfer elastic strand (97) which is at least one elastic strand superposing the front portion (FC) as well as the pair of main bonds (MB). The transfer elastic strand (97) may be an elastic strand having elastic activity across the entire transverse dimension of the front elastic belt, or an elastic strand having an elastic cut window in the transverse center. The elastic cut window may be adjusted such that the transfer elastic strand (97) does not superpose inner cuff elastic strands in the front portion. Whatever the superposing dimension with the front portion (FC), the transfer elastic strand (97) superposes the pair of main bonds (MB) in order to transfer the contracting force to the absorbent main body (38) between the front elastic belt (84) and the front portion (FC). The transfer elastic strand (97) may be one elastic strand, or two elastic strands, or from about 2 to about 15 elastic strands, or from about 3 to about 12 elastic strands, and may have a density of from about 200dtex to about 1100dtex, or from about 300dtex to about 1000dtex, and disposed in an elongation of from about 100% to about 400%, or from about 150% to about 300%. The garment facing side of the absorbent main body (38) attached to the front elastic belt (84) holds tension provided by the combination of transfer elastic strand (97) when contracted upon wear and thus pulled away from the wearer, while the tension provided by the inner cuff elastic strands (35) are transferred to the front portion (FC) to raise the front portion (FC) from the topsheet (24) and against the wearer. The dimensions of the composite isolation sheet (CIS) and the tension provided by the inner cuff elastic strands (35) and the transfer elastic strand (97) are adjusted so that, upon wear, the front portion (FC) thus raised from the topsheet (24) does not cover the urethral orifice of the wearer. The composite isolation sheet (CIS) of the present invention is devoid of elasticity along the pair of front opening edges (102, 103). Even though there is such absence of elasticity around certain portions of the opening of the composite isolation sheet (CIS), there may be provided sufficient tension by the combination of the inner cuff elastic strands (35) and the transfer elastic strand (97) to provide void space between the front portion (FC) and the remainder of the absorbent main body (38). Absence of elasticity in the front opening edges (102, 103) prevents irritation to the wearer's genital area.

Further, such configuration of the front portion (FC) being devoid of elastic strands in the front opening edges (102, 103), and preferably the front portion (FC) being devoid of any elastic strands, enables providing elasticity generally parallel to the longitudinal axis (LX) of the absorbent main body. Such configuration of the composite isolation sheet (CIS) of the present invention is advantageous in that, when the machine direction for manufacturing the absorbent main body matches the longitudinal direction, any elastic body to be disposed on the composite isolation sheet (CIS) may also be advanced in the machine direction for attachment.

Referring to Figures 4A and 6A-6B, the front elastic belt (84) may comprise a fold over portion (93) which is an extension of the outer sheet (92) or the inner sheet (94) folded over the waist opening, wherein the fold over portion may be bonded by a fold over bond (93B), to the absorbent main body (38), or bonded to the front portion (FC). The fold over bond (93B) may extend transversely at least between the side edges of the inner cuff. The fold over bond (93B) may extend the entire transverse dimension of the front elastic belt (84). The fold over bond (93B) and the auxiliary bond may be superposed with each other in the thickness direction. By having the fold over bond (93B) and the auxiliary bond (AB) superposed with each other, this forms a barrier which prevents exudates from flowing into the front opening beyond the absorbent main body (38).

Referring to Figure 4A, the front elastic belt (84) may be divided into multiple zones spanning in the transverse direction and defined by its location from the distal edge (88) to the proximal edge (90) relative to the percentage of the seam length LS wherein the distal edge (88) is considered 0% and the proximal edge (90) is considered 100%. The front elastic belt may comprise a waist zone (202) located at from 0% to 25%, a distal tummy zone (204) located at 25% to 50%, a proximal tummy zone (206) located at from 50% to 85%, and a leg zone (208) located at 85% to 100% of the side seam length LS, wherein the proximal tummy zone has a tensile stress of at least 50% higher than any other zone. The front longitudinal end point (101) or the front portion (FC) may superpose the proximal tummy zone. By superposing the front longitudinal end point (101) with the proximal tummy zone having relatively high tensile stress, this may help pull away the proximal tummy zone from the wearer to provide void space between the front portion (FC) and the remainder of the front elastic belt (84).

### Front Portion and Front Leg Elastic Zone

Referring to Figure 2C, the absorbent article (20) of the present invention may be the uni-body type pant comprising an elastic cover (40) forming the garment facing surface of the entire article having a front region (26) and a back region (28) extending transversely between the side seams (32), and a crotch region (30) positioned longitudinally between the front and back regions (26, 28). The front and back regions of the elastic cover (40) are considered the front elastic belt (84) and the back elastic belt (86). For the uni-body type pant, transverse-running elastic members (96) may be disposed in the front and back elastic belts (84, 86) as well as in the crotch region (30) in the vicinity of the back region (28). By having such additional transverse-running elastic members (96) in the vicinity of the back region (28), this may help fit of the elastic cover (40) against the buttock of the wearer.

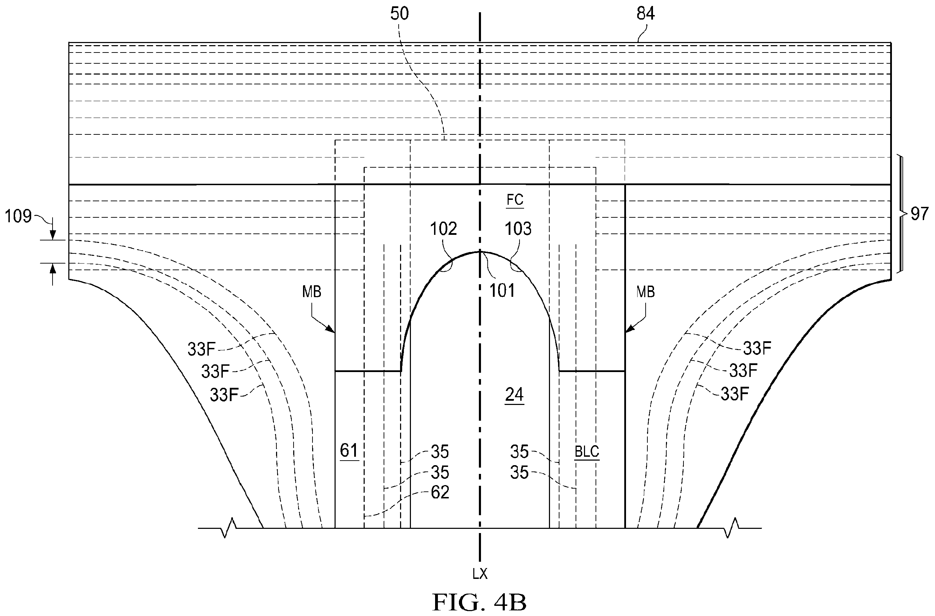

Referring to Figures 2C and 4B, in addition to elastic members (96) running in the transverse direction in the front and back elastic belts (84, 86), there may be elastic members extending from the front elastic belt (84) into the crotch region (30). Elastic bodies which extend into the crotch region (30) generally along the leg openings are defined leg elastic bodies (33) herein. A plurality of leg elastic bodies (33F, 33B) may extend from the proximal edges of the front and back belt regions (84, 86) extending into the crotch region (30) to impart elasticity along the leg opening (LO). The leg elastic bodies may comprise a plurality of front leg elastic bodies (33F) joined between the outer sheet (92) and the inner sheet (94) and extending from the left front region through the crotch region (30) to the right front region, and a plurality of back leg elastic bodies (33B) joined between the outer sheet (92) and the inner sheet (94) and extending from the left back region through the crotch region (30) to the right back region.

Referring to Figures 2C and 4B, the leg elastic bodies may comprise a plurality of front leg elastic bodies (33F) joined between the outer sheet (92) and the inner sheet (94) and extending from the left front region along the leg opening and, at a position approximately in the longitudinal center of the article, extend transversely through the crotch region (30) to the right front region. There may be from about 2 to about 5 front leg elastic bodies (33F) disposed on the elastic cover (40). Each front leg elastic body (33F) may be provided in a density of from about 300dtex to about 1000dtex, and disposed at an elongation of from about 120% to about 400%. The front leg elastic bodies (33F) may all be provided in the same dtex and elongation, or have an increase of dtex and/or elongation toward the inward disposed elastic body. The front leg elastic bodies (33F) may be introduced in the elastic cover (40) such that at any region where the front leg elastic bodies (33F) are disposed, the front leg elastic bodies (33F) may have a spacing of at least about 3mm between each other.

Referring to Figure 4B, the zone matching the location of the front leg elastic bodies at the side seam and spanning in the transverse direction is considered a front leg elastic zone (109). The front longitudinal end point (101) may superpose the front leg elastic zone (109). By superposing the front longitudinal end point (101) with the front leg elastic zone (109) having relatively high tensile stress, this may help pull away garment facing side of the absorbent main body (38) away from the wearer to provide void space between the front portion (FC) and the remainder of the absorbent main body (38).

### Back Waist Guard

Referring to Figure 2B, the absorbent article of the present invention may further comprise a back waist guard (WGP) on the back elastic belt (86). Similar to the front elastic belt (84), the back elastic belt (86) may also be divided into multiple zones spanning in the transverse direction and defined by its location from the distal edge (88) to the proximal edge (90) relative to the percentage of the seam length LS wherein the distal edge (88) is considered 0% and the proximal edge (90) is considered 100%. In the embodiment of Figure 2B, the back elastic belt (86) has a greater longitudinal dimension than that of the front elastic belt (84), thus the front and back elastic belts are seamed together with the distal edges (88) aligned, wherein the proximal portion of the back elastic belt (86) may be left unseamed to form a buttock cover. Thus, the seam length LS has a shorter longitudinal dimension than the back elastic belt (86). The back waist guard (WGP) may be positioned at a zone located at from about 5% to about 50% of LS, or from about 10% to about 50% of LS, wherein the back waist guard (WGP) is formed by at least one of the sheets forming the back elastic belt (86), for example the outer sheet fold over portion (93).

The back waist guard (WGP) is a separate part from the composite isolation sheet (CIS), however may cooperate with the back portion (BC) in order to provide additional protection on the back side. Without being bound by theory, by providing the back waist guard (WGP) in such dimension relative to LS on the back elastic belt (86), the back side is effectively covered towards the waist opening, so that fluid exudate such as urine, runny fecal matter or blood, may be effectively drawn into the pocket, even when the wearer is in active movement.

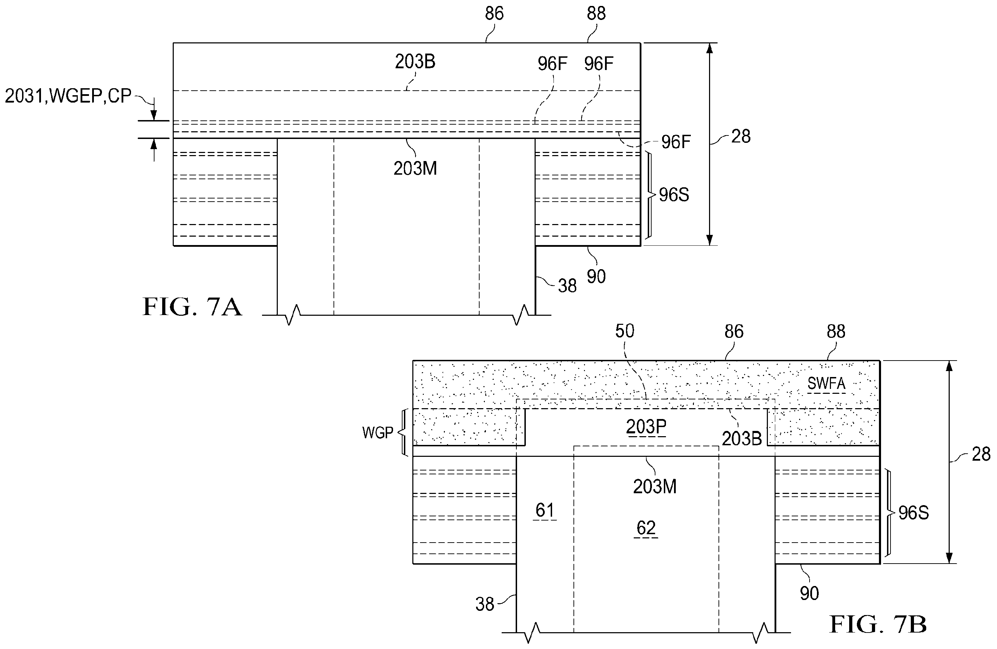

Referring to Figures 7A-7C, the back waist guard (WGP) may be made by an extension of the outer sheet (92), which extension of the outer sheet (92) is folded inwardly 2 times in order to encompass a first elastic member (96F) and further to provide the back waist guard (WGP) in the intended position relative to the side seam length LS. As a result of the 2 folds, the first elastic member (96F) is sandwiched between the first outer sheet fold (931) and the second outer sheet fold (932), wherein the first outer sheet fold (931) constitutes the garment facing side of the back waist guard (WGP), and the second outer sheet fold (932) constitutes the wearer facing surface. Referring to Figures 7B-7C, a certain longitudinal dimension from the distal edge of the second inner sheet fold (932) is bonded to the remainder of the belt, such bonding shown as SWFA, however, leaving some longitudinal dimension unbonded. The position provided most distal of the unbonded region is the closed base line (203B), and the common proximal edge of the first outer sheet fold (931) and second outer sheet fold (932) is the open edge (203M). The back waist guard (WGP) is defined as having a longitudinal dimension spanning from the closed base line (203B) and the open edge (203M), and having a transverse dimension matching the width of the belt to which it is provided. The left and right transverse edges of the back waist guard (WGP) are also bonded to the remainder of the belt, such bonding shown as SWFA. The unbonded region thus provided is the pocket (203P). The bondings provided to define the area of the pocket (203P) are so configured to provide the pocket (203P) superposing the backsheet (25), optionally completely superposing the backsheet (25). There may be other elastic bodies (96S) introduced between the inner sheet (94) and the outer sheet (92).

Referring to Figures 7A-7B, the first elastic member (96F) provides the waist guard elastic portion (WGEP), wherein elasticity may be imparted by one or more elastic strands, elastic ribbons, mechanically activated nonwovens, or otherwise. The waist guard elastic portion (WGEP) is defined as having a longitudinal dimension (2031) spanning from the open edge (203M) to the distal edge of the first elastic member (96F). When the first elastic member (96F) is a plurality of elastic strands, the distal edge of the waist guard elastic portion (WGEP) is the position of the elastic strand which is positioned most distal. There may be 2-10 elastic strands, or from 2-5 elastic strands, or 2-8 elastic strands, or 3-4 elastic strands, or 3-8 elastic strands, or 4-6 elastic strands disposed on the waist guard elastic portion. The longitudinal dimension of the waist guard elastic portion (WGEP) is smaller than that of the back waist guard (WGP) such that a pocket (203P) having containment capacity may be provided. The longitudinal dimension of the waist guard elastic portion (WGEP) may be no greater than about 40mm, or no greater than about 35mm, or no greater than about 30mm, or no greater than about 25mm, or from 15mm to about 40mm, or from about 10mm to about 35mm, or from about 10mm to about 25mm.

Referring to Figures 7A-7C, the remainder of the belt superposing the waist guard elastic portion (WGEP) is defined a correlated portion CP. Namely, the correlated portion CP has the same longitudinal dimension (2031) as the waist guard elastic portion (WGEP). The correlated portion CP may be elasticized or may not be elasticized, wherein the tensile stress of the waist guard elastic portion (WGEP) is higher than that of the correlated portion CP. The correlated portion CP may be elasticized wherein the tensile stress of the waist guard elastic portion (WGEP) is at least about 25% higher, or at least about 100% higher than that of the correlated portion CP. Without being bound by theory, such tensile stress difference enables the waist guard elastic portion (WGEP) to be in close contact with the wearer, while pulling away the open edge (203M) from the correlated portion CP, thus opening the pocket (203P) towards the proximal side. Thus, the pocket (203P) is configured to have good containment capacity. The correlated portion CP may have an elastic cut window such that some portion is elasticized, while the transverse center does not interfere with the opening of the pocket (203P). When the waist guard elastic portion (WGEP) is elasticized by disposing a plurality of elastic strands (96F), one elastic strand is disposed from no less than 2mm to 5mm away from the open edge (203M). Placement of the elastic strand on the very edge of the open edge (203M) may be avoided in order to prevent the back waist guard (WGP) causing irritation to the wearer. The waist guard elastic portion (WGEP) may be disposed with a plurality of elastic strands (96F) with a relatively small pitch between each other. The elastic strands (96F) of the waist guard elastic portion (WGEP) may be disposed at a pitch of no more than about 12mm, or no more than about 10mm, or no more than about 6mm, or between about 2mm to about 8mm, or between about 3mm to about 9mm, or between about 2mm to about 6mm.

Referring to Figure 2B wherein the front region comprises a front portion (FC) and the back region comprises a back waist guard (WGP), the different types of protection layers on the front and back may provide effective protection and containment for different type of bodily exudates. Further, in that the front portion (FC) does not extend beyond the absorbent main body (38), the existence of the front portion (FC) does not add additional substrates to the side seam (32) in the front region. Thus, while the existence of the back waist guard (WGP) increases the number of substrates to the side seam (32) in the back region, the number of layers in any portion of the side seam (32) combining those from the front and back elastic belts (84, 86) comprises no more than 7 layers of substrates. Maintaining the layers at the side seam (32) at a low number is advantageous in view of providing a side seam which has homogenous strength along its longitudinal length, which enables sufficient strength while the article is worn, while being easy to tear open upon removal.

### Inner sheet and Outer sheet

The front elastic belt (84) and the back elastic belt (86) of the present invention may each be formed by an inner sheet (94) and an outer sheet (92) having certain relationship with each other for enhancing certain benefits of the front and back elastic belts (84, 86).

When the inner sheet (94) has an inner sheet hydrophilicity and the outer sheet (92) has an outer sheet hydrophilicity, the outer sheet hydrophilicity may be greater than the inner sheet hydrophilicity. Without being bound by theory, it is believed that such gradient of hydrophilicity is advantageous in transporting skin sweat from the inner sheet (94) to the outer sheet (92) and outside the laminate. The inner sheet (94) nonwoven may be inherently hydrophobic. The inner sheet (94) nonwoven may be provided hydrophobicity by treating with hydrophobic melt additives into polymer resin in the fiber making process, or by applying hydrophobic additives after the nonwoven is formed. The outer sheet (92) nonwoven may inherently be hydrophobic, and thus provided relatively more hydrophilic than the inner sheet (94) by treating with hydrophilic melt additives into polymer resin in the fiber making process, or by applying hydrophilic additive after the nonwoven is formed.

The basis weight of the outer sheet (92) and the inner sheet (94) may be adjusted such that the basis weight of the inner sheet (94) is not greater than the basis weight of the outer sheet (92). Thus, the outer sheet (92) may be provided with a soft lofty tactile sense which connotes high quality, while the inner sheet (94) may be kept thinner and conforming to the outer sheet (92), thus saving cost. Further, without being bound by theory, by providing the basis weight relationship as such, it is believed that skin sweat is effectively transported to the outer sheet (92) and outside the laminate, while preventing the transported sweat back to the inner sheet (94).

### Other components

The absorbent article (20) of the present invention may further comprise components that improve leakage prevention, wearability, fit, or aesthetic aspects of the resulting absorbent article.

The absorbent main body (38) may comprise gasketing leg cuffs (34). Referring to Figures 2A-2B, the gasketing leg cuffs (34) may be placed transversely outward relative to the composite isolation sheet (CIS). The gasketing leg cuffs (34) may provide sealing around the thighs of the wearer. Usually each gasketing leg cuff (34) will comprise an elastic element, for example one or more elastic strands (33) comprised in the absorbent main body (38) for example between a nonwoven material and the backsheet (25) in the area of the leg openings. The substrate for making the composite isolation sheet (CIS) may extend transversely and be utilized for forming the gasketing leg cuffs (34).

While not shown, the absorbent main body (38) of the present invention may comprise an outer cover located on the garment-facing side of the backsheet (25). Referring to Figures 1 and 2A-2B, the outer cover may provide a finished appearance for the garment facing surface of the belt-type pant. The outer cover may be made of a soft, non-woven material. The outer cover and the backsheet (25) may be joined together by adhesive or any other suitable material or method. A particularly suitable outer cover is available from Fibertex NiLai Malaysia with tradename A 10160EJ - MALAYSIA and available from FQN Hazlet NJ with tradename SM1104174.

The absorbent core (62) may comprise an intermediate layer between the layer of absorbent material and the backsheet (25). The intermediate layer may be in direct contact with the layer of absorbent material and with the backsheet (25). The intermediate layer may be useful as a masking layer to isolate the superabsorbent polymer particles in the layer of absorbent material from the backsheet (25), thus reducing graininess feeling and improving the tactile properties of the garment-facing side of the article, especially for absorbent core (62) containing a high level of superabsorbent polymer particles. The intermediate layer may also isolate the exudates which have been absorbed in the layer of absorbent material from the garment-facing side of the article, as this may be visually unpleasant to the caregiver. Thus by having an intermediate layer with a relatively high opacity, stains in the layer of absorbent material (e.g. from urine or feces) can be concealed from view, when looking at the backsheet (25) of the absorbent article during use. The hunter opacity in the dry state of the intermediate layer may be at least 25%, or at least 40%, or at least 50%, or at least 70%. The intermediate layer can also help reduce the residual moisture in contact with the backsheet (25), which may lead to cold/wet feeling for the caregiver, or may lead to the wearer mistaking the cold/wet feeling as liquid leaking out of the absorbent article. The intermediate layer may also serve as a temporary reservoir for liquid that had not been absorbed fast enough by the layer of absorbent material.

The parts of the absorbent article (20) of the present invention may be provided with specifically arranged aesthetics which may enhance the existence of the composite isolation sheet (CIS) and its benefit thereof. The topsheet (24) may be provided with a first topography visible from the wearer facing side, and the composite isolation sheet (CIS) may be provided with a second topography visible from the wearer facing side, wherein the first topography and the second topography are discernible with each other. The second topography may be provided on the front portion (FC) and/or the inner cuffs (BLC). The first and second topography may be selected from the group consisting of color, opening, pattern, and combinations thereof, respectively. Opening may be apertures or slits. Pattern may be embossing, quilting, and other visible deformation.

### Whole Article Force Measurement

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at 23 ±2 °C and 50 ±5 % relative humidity.

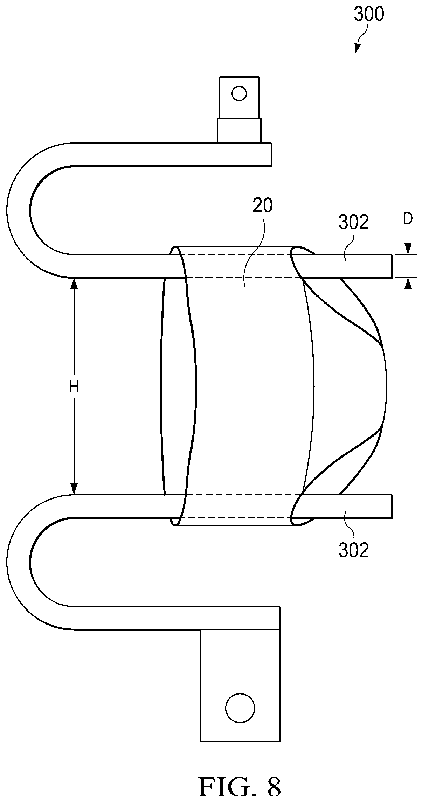

The tensile tester is fitted with hanger-type sample holding fixtures (300) as shown in Figure 8. Each fixture comprises a rigid linear rubber-coated horizontal bar section (302) to prevent sample slippage during testing. The outer bar diameter (including the rubber coating) of the horizontal bar sections is 10.0 mm. The central axes of the horizontal bar sections (302) are configured to remain parallel and in the same vertical plane throughout the test procedure. The gauge circumference is determined by the following equation: $Gauge Circumference = 2 \times \left(H+D+πD/2\right)$ where H is the vertical gap between the horizontal bar sections (302), and D is the outer diameter of the bar.

The instrument is set up to go through the following steps:

| | |
|---|---|
| Crosshead Speed | 254.0mm/min |
| Final Load Point | 19.61 N |
| Hold Time | 0 |
| Number of Cycles | 1 |
| Data Acquisition Rate | 50Hz |

An article (20) sample is inserted onto the upper horizontal bar section (302) so that the bar passes through the waist opening and one leg opening of the article. The crosshead is raised until the specimen hangs above the lower bar and does not touch lower bar (302). The load cell is tared and the crosshead is lowered to enable the lower bar (302) to be inserted through the waist opening and other leg opening without stretching the article. The article is adjusted so that the longitudinal centerline LX of the article is in a horizontal plane halfway between the upper and lower bars (302). The center of the side portion in contact with the bar (302) is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the Initial Gauge Circumference at the same speed. The maximum circumference at 19.6N and the force at 70% of the maximum circumference during the loading segment and unloading segment of the test are recorded.

The maximum circumference at 19.6N is defined as the Full Stretch Circumference (mm). The Full Stretch Circumference (mm) × 0.7 is defined as the 70% Stretch Circumference. The Waist Circumference Force is defined as the force at 70% stretch circumference during the load (extension) segment of the test. Five samples are analyzed and their average are calculated and reported to the nearest 0.01N, respectively.

### Belt Zone Tensile Stress Measurement

The tensile stress (N/m) is calculated by tensile force (N) divided by the specimen width (m). Force may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10% and 90% of capacity of the load cell. The instrument is calibrated according to the manufacturer's instructions. All testing is performed in a room maintained at 23 ±2 °C and 50 ±5 % relative humidity. The instrument is equipped with single line contact grips at least as wide as the test specimen.

To obtain test specimens, the sample article is cut open along the side seams (32), and the elastic cover (40) or the front and belt regions (26, 28) are removed from the absorbent main body (38) by separating the bonding between them, and further the waist guard portion WGP is separated from the belt to which it is attached. Cold Spray may be used, paying attention not to make wrinkles in the belt sections. Care is taken not to spray on any belt elastic member (96F, 96S). The obtained elastic belt samples are severed into zones (202, 204, 206, 208, 2031-WGEP, 2031-CP) according to the present invention with care not to cut any elastic member (96F, 96S). Samples are pre-conditioned at 23 °C ±2 C°and 50% ±5% relative humidity for two hours prior to testing.

The instrument is set up to go through the following steps. Initial Gauge Length is calculated from the Initial Gauge Circumference which is determined during the Whole Article Force Test using separate identical articles, as described above. Initial Gauge Length = 0.5 × Initial Gauge Circumference. The final gauge length is calculated from the Full Stretch Circumference which is determined during the Whole Article Force Test, as described above.

| | |
|---|---|
| Crosshead Speed | 254.0 mm/min |
| Data Acquisition Rate | 50Hz |
| Final Gauge Length | 0.5 × Full Stretch Circumference |
| Hold Time | 0 |
| Number of Cycles | 1 |

One end of the specimen is clamped into the upper clamp and the load is tared. The other end of the specimen is clamped into the lower clamp. Approximately 5 mm of each end of the specimen is behind the contact line of the grip. The test is started and the specimen is extended to the final gauge length at a crosshead speed of 254 mm/min, then immediately returned to the original gauge length at the same speed. The specimen is extended in the article transverse direction during the test. The unload force at 70% of the Final Gauge Length during the unload segments of the test is recorded.

Five articles are analyzed and the unload forces are recorded for each of the zones. The average tensile force (N) is calculated to the nearest 0.01 N for each. The tensile stress for each zone is calculated by the average tensile force (N) divided by the average specimen width (m) and reported to the nearest 0.1 N/m.

### Opacity Method

Opacity is measured using a 0° illumination/45° detection, circumferential optical geometry, spectrophotometer with a computer interface such as the HunterLab LabScan XE running Universal Software (available from Hunter Associates Laboratory Inc., Reston, VA) or equivalent instrument. Instrument calibration and measurements are made using the standard white and black calibration plates provided by the vendor. All testing is performed in a room maintained at 23 ±2 °C and 50 ±2 % relative humidity.

The spectrophotometer is configured for the XYZ color scale, D65 illuminant, 10 ^{o}standard observers, with UV filter set to nominal. The instrument is standardized according to the manufacturer's procedures using the 0.7 inch port size and 0.5 inch area view. After calibration, the software is set to the Y opacity procedure which prompts the operator to cover the sample with either the white or black calibration tile during the measurement.

Articles are pre-conditioned at 23 °C ±2 C° and 50% ±2% relative humidity for two hours prior to testing. To obtain a specimen, the article is stretched flat on a bench, body facing surface upward, and the total longitudinal length of the article is measured. A testing site is selected at the longitudinal midpoint of the article or the part. Using scissors, a test specimen is cut in 60 mm square or as close as possible to 60mm. Any elastic members are removed.

The specimen is placed over the measurement port. The specimen should completely cover the port with the surface corresponding to the wearer-facing surface of the specimen directed toward the port. The specimen is gently extended until taut in its longitudinal direction so that the specimen lies flat against the port plate. Adhesive tape is applied to secure the specimen to the port plate in its extended state for testing. Tape should not cover any portion of the measurement port. The specimen is then covered with the white standard plate. A reading is taken, then the white tile is removed and replaced with the black standard tile without moving the specimen. A second reading is taken, and the opacity is calculated as follows: $Opacity = \left(Y value\left(black backing\right)/Y value\left(white backing\right)\right) \times 100$

Specimens from five identical articles are analyzed and their opacity results recorded. The average opacity is calculated and reported to the nearest 0.01%.

### Air Permeability Test

Air permeability is tested using a TexTest FX3300 Air Permeability Tester (available from Advanced Testing Instruments, Greer, SC) with a custom made 1 cm² circular aperture (also available from Advanced Testing Instruments) or equivalent instrument. The instrument is calibrated according to the manufacturer's procedures. All testing is performed in a room maintained at 23 °C ±2 C° and 50 % ±2 % relative humidity.

The articles are pre-conditioned at 23 °C ±2 C° and 50% ±2% relative humidity for two hours prior to testing. To obtain a specimen, the article is stretched flat on a bench, body facing surface upward, and the total longitudinal length of the article is measured. A testing site is selected at the longitudinal midpoint of the article or the part. Using scissors, a test specimen is cut in 30 mm square or as close as possible to 30 mm. Any elastic members are removed.

The specimen is centered over the measurement port. The specimen should completely cover the port with the surface corresponding to the wearer-facing surface of the specimen directed toward the port. The specimen is gently extended in its longitudinal direction until taut so that the specimen lies flat across the port. Adhesive tape is applied to secure the specimen across the port in its extended state for testing. Tape should not cover any portion of the measurement port. The test pressure is set to allow air to pass through the specimen. For non-woven specimen the pressure is set for 125 Pa and for specimen containing films 2125 Pa is used. The sample ring is closed and the measuring range is adjusted until the range indicator shows green to indicate that the measurement is within the accepted limits of the instrument. The air permeability is recorded to the nearest 0.1 m3/m2/min.

### Hydrostatic Head Test

Hydrostatic head is tested using a TexTest FX3000 Hydrostatic Head Tester (available from Advanced Testing Instruments, Greer, SC) with a custom made 1.5 cm2 circular measurement port (also available from Advanced Testing Instruments). Two annular sleeve rings, the same dimensions as the gaskets around the measurement ports, are cut from the standard protective sleeves for fine nonwovens (part FX3000-NWH, available from Advanced Testing Instruments). The sleeve rings are then adhered with two-sided adhesive tape to the sample facing surfaces of the upper and lower gaskets of the TexTest instrument to protect the specimen during clamping. Standardize the instrument according to the manufacturer's procedures. All testing is performed in a room maintained at about 23 °C ±2 C° and about 50 % ±2 % relative humidity.

Precondition the articles at about 23 °C ±2 C° and about 50% ±2% relative humidity for two hours prior to testing. To obtain a specimen, lay the article stretched flat on a bench, body facing surface upward, and measure the total longitudinal length of the article. A testing site is selected at the longitudinal midpoint of the article or the part. Using scissors, a test specimen is cut in 70 mm square or as close as possible to 70 mm. Any elastic members are removed.

Place the specimen centered over the port of the upper test head. The specimen should completely cover the port with the surface corresponding to the garment-facing surface of the specimen directed toward the port (wearer-facing surface will then be facing the water). Gently extend the specimen taut in its longitudinal direction so that the specimen lies flat against the upper test plate. Adhesive tape is applied to secure the specimen to the test plate in its extended state for testing. Tape should not cover any portion of the measurement port.

Fill the TexTest syringe with distilled water, adding the water through the measurement port of the lower test plate. The water level should be filled to the top of the lower gasket. Mount the upper test head onto the instrument and lower the test head to make a seal around the specimen. The test speed is set to 3 mbar/min for samples that have a hydrostatic head of 50 mbar or less and a speed of 60 mbar/min for samples with a hydrostatic head above 50 mbar. Start the test and observe the specimen surface to detect water droplets penetrating the surface. The test is terminated when one drop is detected on the surface of the specimen or the pressure exceeds 200 mbar. Record the pressure to the nearest 0.5 mbar or record as >200 mbar if there was no penetration detected.

A total of five identical articles are analyzed and their hydrostatic head results recorded. Calculate and report the average hydrostatic head report to the nearest 0.1 mbar.

### EXAMPLES

Example 1 and Comparative Examples 1-2 were obtained as such.

Comparative Example 1: "Pampers Ichiban Pants Size 5" diapers Lot #20221104 purchased from the PRC market in 2022.

Comparative Example 2: "GooN Premium Nature Pants" supplied by Daio Paper with Lot #20220828 purchased from the PRC market in 2022.

Example 1: "Pampers Ichiban Pants Size 5" diapers Lot #20230317 purchased from the PRC market in 2023 and further modified to replace the composite isolation sheet so that it has the front portion (FC) and inner cuff (BLC) configurations of Figure 4A, however, with no back portion (inner cuff extending to the back end of the absorbent main body), and dimensions as in Table 1 below.

Further, Example 1 and Comparative Example 1-2 were subject to a consumer acceptance test as detailed below, and leakage prevention also reported in Table 1 below.

### Consumer Acceptance Test

Example 1, Comparative example 1 and Comparative example 2, were placed in a consumer test among 29 caregivers with babies wearing Size 5 Pant diapers. The panelists were caregivers of Chinese Size 5 (XL-size) wearers of age 0-36 months, all boys, and experienced at least 3 times or above urine leakage when baby is at sleep. The panelists were given enough samples of Example 1, Comparative example 1 and Comparative example 2 to use each Example for 5 days. The 29 panelists were grouped into 6 groups and with each group using the Example rotation plan as in Table 1.

**Table 1**

| Panelist | 1-5 | 6-10 | 11-15 | 16-20 | 21-25 | 26-29 |
|---|---|---|---|---|---|---|
| Product 1 | Comparative Example 1 | Comparative Example 1 | Example 1 | Example 1 | Comparative Example 2 | Comparative Example 2 |
| Product 2 | Example 1 | Comparative Example 2 | Comparative Example 1 | Comparative Example 2 | Comparative Example 1 | Example 1 |
| Product 3 | Comparative Example 2 | Example 1 | Comparative Example 2 | Comparative Example 1 | Example 1 | Comparative Example 1 |

During the 15 days usage, each panelist filled a diary sheet with a question asking whether each sample they used had urine leakage or not. The urine leakage % of each tested sample was calculated as the leaked sample number divided by total sample number used by all the panelists. The total number of used samples are different by Example due to natural variation of consumer usage habits. As the result, the urine leakage % of each Example was as summarized in Table 2.

**Table 2**

| Dimension | Example 1 | Comparative Example 1 | Comparative Example 2 |
|---|---|---|---|
| F1 (mm) | 114 | 0 | 0 |
| F2 (mm) | 64 | 0 | 0 |
| T1 (mm) | 40 | 34 | 40 |
| Total used pads | 487 | 549 | 551 |
| Total leaked pads | 22 | 82 | 81 |
| Leakage (%) | 4.5 *) | 14.9 | 14.7 |

| | | | |
|---|---|---|---|
| *) statistically significantly better than Comparative Examples 1 and 2 at 95% confidence. | | | |

The articles of Example 1, when worn, provide the composite isolation sheet raised from the topsheet, while not covering the urethral orifice of the intended wearer. The articles of Example 1 provide good isolation of urine from the wearer's skin, while maintaining the performance of containment and wear comfort of Comparative Example 1. The articles of Example 1 may be manufactured in approximately the same speed as manufacturing Comparative Example 1.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (20) having a longitudinal direction and a transverse direction comprising a front elastic belt (84), a back elastic belt (86), and a crotch region (30), wherein the front and back elastic belts are connected with a pair of side seams to form a waist opening and a pair of leg openings; the crotch region extending longitudinally between the front elastic belt and the back elastic belt; the absorbent article comprising an absorbent main body (38) extending the entire longitudinal dimension of the crotch region and further extending partly into each of the front region and the back region, the absorbent main body comprising:
1) a water permeable topsheet (24),
2) a water impermeable backsheet (25),
3) an absorbent core (62) disposed between the topsheet and the backsheet, and
4) a composite isolation sheet (CIS) comprising:
a) a pair of inner cuffs (BLC) having a pair of side edges which are elasticized in the longitudinal direction with an inner cuff elastic strand (35), and
b) a front portion (FC), the front portion having a front longitudinal end point in the front region and a pair of front opening edges (102, 103), wherein the front opening edges (102, 103) are free of elastic elements, wherein the front portion has a side longitudinal dimension F1, and a center longitudinal dimension F2, and wherein F2 is from about 30% to about 85% of F1, wherein the composite isolation sheet is bonded to the wearer facing side of the topsheet by a pair of main bonds extending in the longitudinal direction and by an auxiliary bond extending in the transverse direction between the main bonds,
the front elastic belt (84) formed by an outer sheet (92), an inner sheet (94), and a plurality of elastic strands extending in the transverse direction and sandwiched between the outer sheet and the inner sheet, the front elastic belt comprising a transfer elastic strand (97) which is at least one elastic strand superposing the front portion as well as the pair of main bonds (MB),
wherein the absorbent core does not extend to the transverse edges beyond the main bonds, and does not extend to the front end beyond the auxiliary bond (AB).

2. The absorbent article of Claim 1, wherein the inner cuff elastic strand extends into the front portion (FC).

3. The absorbent article of Claim 1, wherein the transfer elastic strand (97) does not superpose the inner cuff elastic strands in the front portion.

4. The absorbent article of Claim 1, wherein each inner cuff has a transverse dimension T1, wherein T1 (mm) and F1 (mm) have the relationship of: $F 1 \geq 248.14 - 4.66 \times T 1 + 0.34 \times {\left(T1-40.79\right)}^{2}$

5. The absorbent article of Claim 1, wherein the main bond is a bond utilizing pressure and heat.

6. The absorbent article of Claim 1, wherein the front opening edges (102, 103) comprise a concave portion against the front portion.

7. The absorbent article of Claim 1, wherein the front opening edges (102, 103) form a portion of a sinusoidal curve.

8. The absorbent article of Claim 1, wherein the front opening edges are a series of straight lines.

9. The absorbent article of Claim 1, wherein at least one of the outer sheet and the inner sheet is extended and folded over the waist opening to form a fold over portion, and wherein the fold over portion is bonded by a fold over bond to the absorbent main body or to the front portion.

10. The absorbent article of Claim 9, wherein the fold over bond and the auxiliary bond are superposed with each other in the thickness direction.

11. The absorbent article of Claim 9, wherein the composite isolation sheet further comprises a back portion (BC), the back portion having a back longitudinal end point and a pair of back opening edges, wherein the back opening edges are free of elastic elements, wherein the back portion has a side longitudinal dimension B1, and a center longitudinal dimension B2, and wherein B2 is less than about 95% of B1.

12. The absorbent article of Claim 1, wherein the entirety of the length of the transverse edges of the front elastic belt is seamed with a certain length of the transverse edges of the back elastic belt to define a side seam length LS, the front elastic belt divided into multiple zones spanning in the transverse direction and defined by its location from the distal edge to the proximal edge relative to the percentage of the seam length LS wherein the distal edge is considered 0% and the proximal edge is considered 100%, wherein the front elastic belt comprises a waist zone located at from 0% to 25%, a distal tummy zone located at 25% to 50%, a proximal tummy zone located at from 50% to 85%, and a leg zone located at 85% to 100% of the side seam length LS, respectively, wherein the proximal tummy zone has a tensile stress of at least 50% higher than any other zone, and wherein the front longitudinal end point superposes the proximal tummy zone.

13. The absorbent article of Claim 1, comprising a plurality of front leg elastic bodies (33F) joined between the outer sheet and the inner sheet and extending from one side seam of the front elastic belt through the crotch region to the other side seam of the front elastic belt, wherein the zone matching the location of the front leg elastic bodies at the side seam and spanning in the transverse direction form a front leg elastic zone (109), and wherein the front longitudinal end point superposes the front leg elastic zone.

14. The absorbent article of Claim 1, wherein any portion of the side seam comprises no more than 7 layers of substrates.

15. The absorbent article of Claim 1, comprising a back waist guard formed by at least one of the sheets forming the back elastic belt, wherein the back waist guard comprises a pocket (203P).

## Patentansprüche

1. Absorptionsartikel (20), der eine Längsrichtung und eine Querrichtung aufweist, umfassend einen vorderseitigen Gummibandbund (84), einen rückseitigen Gummibandbund (86) und einen Schrittbereich (30), wobei der vorderseitige und der rückseitige Gummibandbund mit einem Paar von Seitennähten verbunden sind, um eine Taillenöffnung und ein Paar von Beinöffnungen auszubilden; wobei sich der Schrittbereich zwischen dem vorderseitigen Gummibandbund und dem rückseitigen Gummibandbund längs erstreckt; der Absorptionsartikel umfassend einen Absorptionsgrundkörper (38), der sich über die gesamte Längsabmessung des Schrittbereichs erstreckt und sich ferner teilweise jeweils in den vorderseitigen Bereich und den rückseitigen Bereich erstreckt, der Absorptionsgrundkörper umfassend:
1) eine wasserdurchlässige Oberschicht (24),
2) eine wasserundurchlässige Unterschicht (25),
3) einen Absorptionskern (62), der zwischen der Oberschicht und der Unterschicht eingerichtet ist, und
4) eine Verbundisolationslage (CIS), umfassend:
a) ein Paar von Innenbündchen (BLC), die ein Paar von Seitenrändern aufweisen, die in der Längsrichtung mit einem Gummibandstrang (35) des Innenbündchens elastifiziert sind, und
b) einen vorderseitigen Abschnitt (FC), wobei der vorderseitige Abschnitt einen vorderseitigen Längsendpunkt in dem vorderen Bereich und ein Paar von vorderseitigen Öffnungsrändern (102, 103) aufweist, wobei die vorderseitigen Öffnungsränder (102, 103) frei von Gummibandelementen sind, wobei der vorderseitige Abschnitt eine Seitenlängsabmessung F1 und eine Mittenlängsabmessung F2 aufweist und wobei F2 von etwa 30 % bis etwa 85 % von F1 beträgt,
wobei die Verbundisolationslage mit der trägerseitigen Seite der Oberschicht durch ein Paar von Hauptbindungen, die sich in der Längsrichtung erstrecken, und durch eine Hilfsbindung, die sich in der Querrichtung zwischen den Hauptbindungen erstreckt, gebunden ist,
wobei der vorderseitige Gummibandbund (84) durch eine Außenlage (92), eine Innenlage (94) und eine Vielzahl von Gummibandsträngen ausgebildet ist, die sich in der Querrichtung erstrecken und zwischen der Außenlage und der Innenlage beidseitig bedeckt sind, der vorderseitige Gummibandbund umfassend einen Übertragungsgummibandstrang (97), der wenigstens ein Gummibandstrang ist, der über dem vorderseitigen Abschnitt und dem Paar von Hauptbindungen (MB) angeordnet ist,
wobei sich der Absorptionskern nicht bis zu den Querrändern über die Hauptbindungen hinaus erstreckt und sich nicht bis zum vorderseitigen Ende über die Hilfsbindung (AB) hinaus erstreckt.

2. Absorptionsartikel nach Anspruch 1, wobei sich der Gummibandstrang des Innenbündchens in den vorderseitigen Abschnitt (FC) erstreckt.

3. Absorptionsartikel nach Anspruch 1, wobei der Übertragungsgummibandstrang (97) nicht über den Gummibandsträngen des Innenbündchens in dem vorderseitigen Abschnitt angeordnet ist.

4. Absorptionsartikel nach Anspruch 1, wobei jedes Innenbündchen eine Querabmessung T1 aufweist, wobei T1 (mm) und F1 (mm) die Beziehung aufweisen: $F 1 \geq 248 , 14 - 4 , 66 \times T 1 + 0 , 34 \times {\left(T1-40,79\right)}^{2}$

5. Absorptionsartikel nach Anspruch 1, wobei die Hauptbindung eine Bindung ist, die Druck und Wärme nutzt.

6. Absorptionsartikel nach Anspruch 1, wobei die vorderseitigen Öffnungsränder (102, 103) einen konkaven Abschnitt gegen den vorderseitigen Abschnitt umfassen.

7. Absorptionsartikel nach Anspruch 1, wobei die vorderseitigen Öffnungsränder (102, 103) einen Abschnitt einer sinusförmigen Kurve ausbilden.

8. Absorptionsartikel nach Anspruch 1, wobei die vorderseitigen Öffnungsränder eine Reihe von geradkettigen Linien sind.

9. Absorptionsartikel nach Anspruch 1, wobei wenigstens eine der Außenlage und der Innenlage verlängert und über die Taillenöffnung gefaltet ist, um einen Faltungsabschnitt auszubilden, und wobei der Faltungsabschnitt durch eine Faltungsbindung mit dem Absorptionsgrundkörper oder mit dem vorderseitigen Abschnitt gebunden ist.

10. Absorptionsartikel nach Anspruch 9, wobei die Faltungsbindung und die Hilfsbindung in der Dickenrichtung übereinander angeordnet sind.

11. Absorptionsartikel nach Anspruch 9, wobei die Verbundisolationslage ferner einen rückseitigen Abschnitt (BC) umfasst, wobei der rückseitige Abschnitt einen rückseitigen Längsendpunkt und ein Paar von rückseitigen Öffnungsrändern aufweist, wobei die rückseitigen Öffnungsränder frei von Gummibandelementen sind, wobei der rückseitige Abschnitt eine Seitenlängsabmessung B1 und eine Mittenlängsabmessung B2 aufweist und wobei B2 kleiner als etwa 95 % von B1 ist.

12. Absorptionsartikel nach Anspruch 1, wobei die Gesamtheit der Länge der Querränder des vorderseitigen Gummibandbundes mit einer gewissen Länge der Querränder des rückseitigen Gummibandbundes genäht ist, um eine Seitennahtlänge LS zu definieren, wobei der vorderseitige Gummibandbund in mehrere Zonen unterteilt ist, die sich in der Querrichtung spannt und durch ihre Position von dem distalen Rand zum dem proximalen Rand relativ zu dem Prozentsatz der Nahtlänge LS definiert sind, wobei der distale Rand als 0 % betrachtet wird und der proximale Rand als 100 % betrachtet wird, wobei der vorderseitige Gummibandbund eine Taillenzone, die sich bei von 0 % bis 25 % befindet, eine distale Bauchzone, die sich bei 25 % bis 50 % befindet, eine proximale Bauchzone, die sich bei von 50 % bis 85 % befindet, beziehungsweise eine Beinzone, die sich bei 85 % bis 100 % der Seitennahtlänge LS befindet, umfasst, wobei die proximale Bauchzone eine Zugspannung von wenigstens 50 % höher als eine beliebige andere Zone aufweist und wobei der vorderseitige Längsendpunkt über der proximalen Bauchzone angeordnet ist.

13. Absorptionsartikel nach Anspruch 1, umfassend eine Vielzahl von vorderseitigen Beingummibandkörpern (33F), die zwischen der Außenlage und der Innenlage aneinandergefügt sind und sich von einer Seitennaht des vorderseitigen Gummibandbundes durch den Schrittbereich zu der anderen Seitennaht des vorderseitigen Gummibandbundes erstrecken, wobei die Zone, die mit der Position der vorderseitigen Beingummibandkörper an der Seitennaht übereinstimmt und sich in der Querrichtung spannt, eine vorderseitige Beingummibandzone (109) ausbildet und wobei der vorderseitige Längsendpunkt über der vorderseitige Beingummibandzone angeordnet ist.

14. Absorptionsartikel nach Anspruch 1, wobei ein beliebiger Abschnitt der Seitennaht nicht mehr als 7 Schichten von Substraten umfasst.

15. Absorptionsartikel nach Anspruch 1, umfassend einen rückseitigen Taillenschutz, der durch wenigstens eine der Lagen ausgebildet ist, die den rückseitigen Gummibandbund ausbilden, wobei der rückseitige Taillenschutz eine Tasche (203P) umfasst.

## Revendications

1. Article absorbant (20) ayant une direction longitudinale et une direction transversale comprenant une ceinture élastique avant (84), une ceinture élastique arrière (86) et une région d'entrejambe (30), dans lequel les ceintures élastiques avant et arrière sont reliées par une paire de coutures latérales pour former une ouverture de taille et une paire d'ouvertures de jambe ; la région d'entrejambe s'étendant longitudinalement entre la ceinture élastique avant et la ceinture élastique arrière ; l'article absorbant comprenant un corps principal absorbant (38) s'étendant sur toute la dimension longitudinale de la région d'entrejambe et s'étendant en outre en partie dans chacune de la région avant et de la région arrière, le corps principal absorbant comprenant :
1) une feuille de dessus perméables à l'eau (24),
2) une feuille de fond imperméable à l'eau (25),
3) une âme absorbante (62) disposée entre la feuille de dessus et la feuille de fond, et
4) une feuille d'isolation composite (CIS) comprenant :
a) une paire de manchettes internes (BLC) ayant une paire de bords latéraux qui sont rendus élastiques dans la direction longitudinale avec un brin élastique de manchette interne (35), et
b) une partie avant (FC), la partie avant ayant un point d'extrémité longitudinal avant dans la région avant et une paire de bords d'ouverture avant (102, 103), dans lequel les bords d'ouverture avant (102, 103) sont dépourvus d'éléments élastiques, dans lequel la partie avant a une dimension longitudinale latérale F1 et une dimension longitudinale centrale F2 et dans lequel F2 est d'environ 30 % à environ 85 % de F1,
dans lequel la feuille d'isolation composite est liée au côté faisant face au porteur de la feuille de dessus par une paire de liaisons principales s'étendant dans la direction longitudinale et par une liaison auxiliaire s'étendant dans la direction transversale entre les liaisons principales,
la ceinture élastique avant (84) étant formée par une feuille externe (92), une feuille interne (94) et une pluralité de brins élastiques s'étendant dans la direction transversale et intercalés entre la feuille externe et la feuille interne, la ceinture élastique avant comprenant un brin élastique de transfert (97) qui est au moins un brin élastique superposé à la partie avant ainsi que la paire de liaisons principales (MB),
dans lequel l'âme absorbante ne s'étend pas vers les bords transversaux au-delà des liaisons principales et ne s'étend pas vers l'extrémité avant au-delà de la liaison auxiliaire (AB).

2. Article absorbant selon la revendication 1, dans lequel le brin élastique de manchette interne s'étend jusque dans la partie avant (FC).

3. Article absorbant selon la revendication 1, dans lequel le brin élastique de transfert (97) ne se superpose pas aux fils élastiques de manchette interne dans la partie avant.

4. Article absorbant selon la revendication 1, dans lequel chaque manchette interne a une dimension transversale T1,
dans lequel T1 (mm) et F1 (mm) ont la relation de : $F 1 \geq 248 , 14 - 4 , 66 \times T 1 + 0 , 34 \times {\left(T1-40,79\right)}^{2}$

5. Article absorbant selon la revendication 1, dans lequel la liaison principale est une liaison utilisant de la pression et de la chaleur.

6. Article absorbant selon la revendication 1, dans lequel les bords d'ouverture avant (102, 103) comprennent une partie concave contre la partie avant.

7. Article absorbant selon la revendication 1, dans lequel les bords d'ouverture avant (102, 103) forment une partie d'une courbe sinusoïdale.

8. Article absorbant selon la revendication 1, dans lequel les bords d'ouverture avant sont une série de lignes droites.

9. Article absorbant selon la revendication 1, dans lequel au moins l'une parmi la feuille externe et la feuille interne est étendue et pliée sur l'ouverture de taille pour former une partie repliée et dans lequel la partie repliée est liée par une liaison de repli au corps principal absorbant ou à la partie avant.

10. Article absorbant selon la revendication 9, dans lequel la liaison de repli et la liaison auxiliaire sont superposées l'une à l'autre dans la direction d'épaisseur.

11. Article absorbant selon la revendication 9, dans lequel la feuille d'isolation composite comprend en outre une partie arrière (BC), la partie arrière ayant un point d'extrémité longitudinal arrière et une paire de bords d'ouverture arrière, dans lequel les bords d'ouverture arrière sont dépourvus d'éléments élastiques, dans lequel la partie arrière a une dimension longitudinale latérale B1 et une dimension longitudinale centrale B2 et dans lequel B2 est de moins d'environ 95 % de B1.

12. Article absorbant selon la revendication 1, dans lequel la totalité de la longueur des bords transversaux de la ceinture élastique avant est cousue avec une certaine longueur des bords transversaux de la ceinture élastique arrière pour définir une longueur de couture latérale LS, la ceinture élastique avant étant divisée en multiples zones s'étendant sur la direction transversale et définie par son emplacement allant du bord distal au bord proximal par rapport au pourcentage de la longueur de couture LS dans lequel le bord distal est considéré comme 0 % et le bord proximal est considéré comme 100 %, dans lequel la ceinture élastique avant comprend une zone de taille située de 0 % à 25 %, une zone de ventre distale située de 25 % à 50 %, une zone de ventre proximale située de 50 % à 85 %, et une zone de jambe située de 85 % à 100 % de la longueur de couture latérale LS, respectivement, dans lequel la zone proximale de ventre présente une résistance à la traction au moins 50 % supérieure à toute autre zone, et dans lequel le point d'extrémité longitudinal avant se superpose à la zone de ventre proximale.

13. Article absorbant selon la revendication 1, comprenant une pluralité de corps élastiques de jambe avant (33F) joints entre la feuille externe et la feuille interne et s'étendant à partir d'une couture latérale de la ceinture élastique avant à travers la région d'entrejambe jusqu'à l'autre couture latérale de la ceinture élastique avant, dans lequel la zone correspondant avec l'emplacement des corps élastiques de jambe avant au niveau de la couture latérale et s'étendant sur la direction transversale forme une zone élastique de jambe avant (109) et dans lequel le point d'extrémité longitudinal avant se superpose à la zone élastique de jambe avant.

14. Article absorbant selon la revendication 1, dans lequel une partie quelconque de la couture latérale ne comprend pas plus de 7 couches de substrats.

15. Article absorbant selon la revendication 1, comprenant une protection de taille arrière formée par au moins l'une des feuilles formant la ceinture élastique arrière, dans lequel la protection de taille arrière comprend une poche (203P).
